# EUROPEAN PATENT SPECIFICATION

(11) **EP 3 397 332 B1**
(45) Date of publication and mention of the grant of the patent: **07.04.2021**
(21) Application number: 16820130.9
(22) Date of filing: 14.12.2016
(51) Int. Cl.: A61M 16/10, A61M 16/16, A61M 16/14

(54) **AXIAL FLOW VAPOR TRANSFER CARTRIDGE WITH LARGE DIAMETER FIBERS**
AXIALFLUSSDAMPFTRANSFERTPATRONE MIT FASERN MIT GROSSEM DURCHMESSER
CARTOUCHE DE TRANSFERT DE VAPEUR À FLUX AXIAL AYANT DES FIBRES DE GRAND DIAMÈTRE

(30) Priority: 29.12.2015 US 201514983225
(43) Date of publication of application: 07.11.2018
(73) Proprietor: Vapotherm, Inc., Exeter, NH 03833 (US)
(72) Inventor: LEONARD, Scott, A., Bedford NH 03110 (US)
(74) Representative: Rashid, Jeremy Suhail
(86) International application number: PCT/US2016/066652
(87) International publication number: WO 2017/116713

(56) References cited:
- EP-A2- 1 281 413
- WO-A1-2015/114328
- WO-A1-2016/161092
- WO-A2-2016/109294
- US-A- 4 146 597
- US-A- 4 155 961
- US-A- 4 367 734
- US-A- 5 996 976
- US-A1- 2004 254 524
- US-A1- 2010 059 053

## Description

### Background

Patients with respiratory ailments may be treated with respiratory assist devices, for example, devices that deliver supplemental breathing gas to a patient. Such devices may deliver gas to a patient using high flow therapy (HFT). HFT devices deliver a high flow rate of breathing gas to a patient via an interface such as a nasal cannula to increase a patient's fraction of inspired oxygen (FiO2), decrease a patient's work of breathing, or to do both. One category of these devices uses membranes to humidify the gasses delivered to the patient. Such systems require gas to be delivered at a particular pressure in order to compensate for the pressure drop experienced as gas moves through the system.

Unfortunately, in some settings access to high pressure gas needed for HFT using conventional systems is not readily available. Additionally, many patients may desire to administer therapy in the comfort of their home or in non-clinical settings.

Moreover, even if a patient has access to a source of high pressure gas or is otherwise able to pressurize gas, the gas needs to be heated and humidified to reduce patient discomfort. A challenge associated with delivering breathing gas via a high-flow system is managing heated and humidified gas that is carried to the patient. During transport of heated and humidified breathing gas, moisture from the heated and humidified breathing gas can condense and form liquid droplets. Condensation in a ventilation circuit presents both clinical and mechanical challenges. The condensate can accumulate in the gas circuit and thus limit flow through the system. Movement of accumulated condensate liquid in the gas circuit into the patient can present a risk of aspiration. Additionally, the condensate can collect and stagnate, posing a biohazard.

One solution to prevent condensation is to heat the tube carrying the humidified breathing gas from a supply unit to a patient interface using a heated water jacket or using a heated wire disposed within the tube. Unfortunately, tubes with heated water jackets can be heavy due to the weight of the water in the tubes. Tubes with heated water jackets also have narrow gas passageways, further increasing the flow resistance of the system. Additionally, tubes with heated water jackets require a pump capable of circulating the heated fluid through the tube, which increases the overall complexity and cost of the respiratory therapy system. Additionally, tubes having heated wires require a connection to an electrical power source, which also adds to the complexity and cost of the respiratory therapy system. The spaces between the wires where the tube is not heated can cause condensation within the tube. Finally, heated wires present in a tube may also pose a safety hazard if the wires overheat.

Placement of the humidifying device proximal to the patient may also decrease the occurrence of collected condensate in supply tubing. Humidification of the breathing gas near to the patient decreases the length of tubing required to transport the humidified breathing gas from the humidifier to the patient and decreases the cumulative surface area of the tubing through which the humidified breathing gas loses heat While the humidified breathing gas retains more heat over the shorter distance to the patient and less condensation is experienced, placement of the humidifier near to the patient is difficult due to the heat emitted by the humidifier, the risk of tangling tubes, wires, etc., and the bum risk associated with moving the heat source (e.g., a hot pot) near the patient, any of which may increase both the danger and/or discomfort experienced by the patient.

US4,155,961 discloses a respiratory air humidifier including a tubular member defining a flow passage for the respiratory air having an inlet at one end and an outlet at the opposite end for the circulation of the respiratory air therethrough. The tubular member comprises a bundle of tubules of U-shape configuration, each tubule having a diameter of less than 300 microns and connected to a water conduit.

### Summary

The invention is defined in the appended claims. Accordingly, disclosed herein are systems, methods, and devices for heating and humidifying a breathing gas that reduces and/or eliminates the dangers and discomforts experienced by a patient. For example, these systems, methods, and devices reduce the pressure drop experienced during operation of conventional humidifiers through the use of large diameter, non-porous membranes. The large diameter membranes allow gas to move easily through the membranes, reducing the amount of pressure needed to move the gas and the resulting pressure drop. By reducing the pressure drop, these systems, methods, and devices may be used in settings (e.g., the home of a patient) without the source of high pressure gas needed in conventional systems, which may greatly increase the comfort and accessibility of these systems, methods, and devices.

In another example, these systems, methods, and devices reduce condensation of the humidified gas by moving the vapor transfer unit proximal to the patient. For example, these systems, methods, and devices deliver gas and fluid to a vapor transfer unit separately in order to reduce rain out and the mechanical and clinical challenges associated with it for the vast majority of the tubing in these systems, methods, and devices. For example, the vapor transfer unit is configured to be positioned proximate to the patient interface, for optimum heating and humidification of breathing gas without condensation of the humidified gas over a long transport length. By transporting the gas and fluid from the capital unit separately, the gas is not exposed to fluid prior to entering the humidifier. As the gas is not humidified until a point proximate to the user, the areas in which condensation may occur is greatly reduced. Moreover, as the humidified gas is now proximate to the user, the length of tubing over which the heated and humidified gas can cool and allow moisture to condense before delivery to the patient is reduced. Thus, by moving the location of vapor transfer proximate to the patient, the amount of condensation that can occur during operation is reduced and the need for systems to control condensation and rain out is reduced.

Furthermore, the systems provide a humidifier with a sleek profile which protects the humidifier from damage during use and reduces the bulk and tangle of tubing near the user. For example, the vapor transfer unit is designed as a cylinder with gas and fluid tubes attached at a port at the end. This places the tubes away from the sides of the vapor transfer unit so that they are less likely to be tangled, kinked, or dislodged, as well as minimizing any obstruction to the user. Further, the fluid tubes transporting heated fluid may be disposed inside the gas tube in order to insulate the fluid during transport from the capital unit, prevent a drop in temperature, and protect the fluid tubes from the tangling or kinking.

As an added benefit, these systems and devices are designed for cost-effective and efficient manufacture. By lowering the cost of manufacture, the cost to the patient for these systems and devices may be reduced. For example, the vapor transfer unit as well as other components (e.g., the large diameter, non-porous membranes) are sized and shaped to be easily extruded for cost-effective and efficient manufacture of the humidifier. Moreover, the axial design limits the number of components that must be fit together and molds that must be used to create the humidifier.

These systems, methods, and devices also allow for a capital unit and/or heating source to be moved away from a patient. For example, as discussed above, because the vapor transfer unit is placed proximal to the patient and the gas travels only a short distance to the patient after being humidified, the risk of condensation and/or rain out prior to humidification is greatly reduced. Accordingly, the capital unit may be moved farther away from the patient as increasing the length the gas travels prior to humidification does not increase the risk of condensation. Furthermore, moving the capital unit farther from the patient allows the capital unit to incorporate more cost-efficient components and/or components that reduce patient discomfort. For example, a blower, as opposed to a source of high pressure gas, may also be used in the system which reduces the noise of the system, provides a better patient experience, and increases accessibility of the humidifier.

These systems, methods, and devices also reduce the biohazard risks to a patient through the use of large diameter, nonporous membranes. For example, by lowering the pressure drop, as discussed above, large diameter membranes allow use of a blower by providing greater ease of gas passage and more surface area through which fluid may absorb. Nonporous membranes have the added benefit of not allowing bacteria to enter the humidified gas delivered to the patient, thus increasing the safety of the patient.

Additionally, systems, methods, and devices allow the vapor transfer unit to be placed proximal to a patient without being in patient contact. By using a water jacket around the humidified gas coming from the vapor transfer unit, the amount of cooling of the gas and condensation is reduced. Patient comfort is also increased as moving the vapor transfer unit away from the body results in a lowered sensation of heat from the vapor transfer unit and an increased range of motion during therapy.

In one aspect, a system for heating and humidifying gases includes a body, extending along a first axis from a first face to a second face, a first lumen at the first face, through which fluid enters the body, a second lumen at the first face through which fluid exits the body, and a nonporous membrane located inside the body and extending from the first face to the second face wherein gas moving through the nonporous membrane is humidified by the fluid.

In some implementations, the first lumen and the second lumen are substantially parallel to the first axis. In some implementations, the first lumen and the second lumen have different lengths. In some implementations, the first lumen includes an aperture allowing the fluid to contact the first nonporous membrane while inside the body. In some implementations, the first nonporous membrane has a diameter of about 0.5 millimeters to 20 millimeters. In some implementations, the inner diameter of the first nonporous membrane may be 0.65 millimeters. In some implementations, the first nonporous membrane is permeable.

In some implementations, the system includes a second nonporous membrane located inside the body and extending from the first face to the second face. The second nonporous membrane is bonded to the first nonporous membrane at the first and second faces. The fluid may move through the system in a first direction and the gas may move in a second direction that is substantially parallel to the first direction. In some implementations, the system also includes a first fluid passageway connecting to the first lumen, through which the fluid is delivered to the body, a second fluid passageway connecting to the second lumen, through which the fluid is removed from the body, and a first gas passageway through which the gas is delivered to the body. The first fluid passageway and the second fluid passageway may be housed within the first gas passageway. In some implementations, the system includes a first end cap connecting the body to the first gas passageway at the first face. The first fluid passageway and the second fluid passageway may pass through the first end cap. In some implementations, the system further includes a second gas passageway which delivers humidified gas from the body to a patient. In some implementations, the system may further include a water jacket located at the second gas passageway. The water jacket warms the humidified gas as the humidified gas is delivered from the body to the patient.

In another aspect, a method for manufacturing a humidifier component comprises inserting a plurality of nonporous membranes into a first lumen of an extruded body, which extends in an axial direction from a first side of the extruded body to a second side of the extruded body. The method also includes bonding the plurality of nonporous membranes together at the first side of the extruded body and at the second side of the extruded body and connecting a first fluid passageway to a second lumen extending in the axial direction from the first side of the extruded body and connecting a second fluid passageway to a third lumen extending in the axial direction from the first side of the extruded body.

In some implementations, the first fluid passageway and the second fluid passageway are housed within a first gas passageway, and the first gas passageway delivers gas to the first lumen. In some implementations, the first lumen opens to an interior area of the extruded body through an interior opening located between the first side and the second side. The interior opening may be created by the first lumen by penetrating a third side of the extruded body and sealing the third side of the extruded body after penetration.

In some implementations, a fourth lumen extends in the axial direction from the first side of the extruded body to the second side of the extruded body without opening to the interior area. In some implementations, bonding the plurality of nonporous membranes together may further comprise fitting a first cap to the first side of the extruded body and a second cap to the second side of the extruded body. Placement of the first cap and the second cap may completely close the first lumen of the extruded body except at the interior opening. Bonding the plurality of nonporous membranes together may additionally comprise injecting a potting material into the extruded body. In some implementations, bonding the plurality of nonporous membranes together may include centrifuging the extruded body to deposit the potting material at the first side of the extruded body adjacent to the first cap and at the second side of the extruded body adjacent to the second cap. In some implementations, the extruded body, first lumen and second lumen comprise a single extrusion. The second lumen may be defined by a first interior passageway and the third lumen may be defined by a second interior passageway. The first interior passageway and the second interior passageway may be housed within the extruded body and bonded to the plurality of nonporous membranes.

The disclosed features may be implemented, in any combination and subcombination (including multiple dependent combinations and subcombinations), with one or more other features described herein. The various features described or illustrated above, including any components thereof, may be combined or integrated in other systems. Moreover, certain features may be omitted or not implemented.

### Brief -description of the drawings

The foregoing and other objects and advantages will be apparent upon consideration of the following detailed description, taken in conjunction with the accompanying drawings, in which like reference characters refer to like parts throughout, and in which:
FIG. 1 shows an illustrative respiratory therapy system;
FIG. 2 shows an illustrative axial flow humidifier for humidifying breathing gas for delivery to a proximal patient;
FIG. 3 shows an illustrative view of the fluid lumens attached to the axial flow humidifier of FIG. 2;
FIG. 4 shows an illustrative cross-sectional view of an axial flow humidifier;
FIG. 5 shows an illustrative view of the axial flow humidifier of FIG. 1 in connection with a patient delivery cannula;
FIG. 6 shows an illustrative axial flow humidifier having fluid flow in a direction not counter to the gas flow;
FIG. 7 shows an illustrative axial flow humidifier having an alternative fluid flow return aperture;
FIG. 8 shows an illustrative axial flow humidifier having extended fluid and gas pathways for use at a distance from the patient;
FIG. 9 shows an illustrative respiratory therapy delivery system including a water jacket;
FIG. 10 shows an illustrative extruded body of an axial flow humidifier;
FIG. 11 shows an illustrative extruded body of an axial flow humidifier having a secondary cut;
FIG. 12 shows an illustrative extruded body of an axial flow humidifier with inserted fiber bundle;
FIG. 13 shows an illustrative extruded body of an axial flow humidifier with installed cap,
FIG. 14 shows an illustrative extruded body of an axial flow humidifier with injected potting material;
FIG. 15 shows an illustrative extruded body of an axial flow humidifier with removed cap;
FIG. 16 shows an illustrative extruded body of an axial flow humidifier having a cut end; and
FIG. 17 shows an illustrative cross-sectional view of an axial flow humidifier having two lumens on one side of the cross-sectional body.

### Detailed Description

To provide an overall understanding of the systems, devices, and methods described herein, certain illustrative embodiments will be described. Although the embodiments and features described herein are specifically described for use in connection with a high flow therapy system, it will be understood that all the components and other features outlined below may be combined with one another in any suitable manner and may be adapted and applied to other types of respiratory therapy and respiratory therapy devices, including low flow oxygen therapy, continuous positive airway pressure therapy (CPAP), mechanical ventilation, oxygen masks, Venturi masks, and tracheotomy masks.

The systems, methods, and devices described herein reduce condensation of humidified gas (e.g., breathable air) by moving the vapor transfer unit proximal to the patient. The vapor transfer unit is configured to be positioned proximate to the patient interface, for optimum heating and humidification of breathing gas without condensation of the humidified gas over a long transport length. By transporting the gas and fluid (e.g., water) from the capital unit separately, the gas is not exposed to water vapor prior to entering the humidifier. As a result, the systems, methods, and devices reduce the distance that heated and humidified breathing gas has to travel before reaching the patient. This reduces the length of tubing over which the heated and humidified gas can cool and allow moisture to condense. Thus, by moving the location of vapor transfer proximate to the patient, the amount of condensation that can occur during operation is reduced and the need for systems to control condensation is reduced. Patient safety is further increased by use of nonporous membranes which do not allow transfer of bacteria from the fluid into the gas flow. Since the gas and fluid are not in contact until they enter the vapor transfer system and then only through the nonporous membrane, this leads to a safer humidified breathing gas provided to the patient.

The systems, methods, and devices described herein also limit the cost and system requirements of the vapor transfer unit. The vapor transfer unit has an axial flow design in which the gas and fluid tubes are attached at an end, which minimizes the bulk of the unit and is also easily extruded. The fiber membranes used in the vapor transfer unit are large diameter fibers which are cost effective and easily extruded in addition to being effective in heating and humidifying the gas flow. Additionally, the system is configured to run with lower pressure, allowing a blower to be used at the capital unit for quieter operation and use in locations without available high pressure air sources.

Furthermore, the systems provide a humidifier with a sleek profile that maximizes patient comfort. The vapor transfer unit is designed as a cylinder with gas and fluid tubes attached at a port at the end and no lateral ports or tubes. This places the tubes away from the sides of the vapor transfer unit so that they are less likely to be tangled, kinked or dislodged. Further, the fluid tubes transporting heated fluid may be disposed inside the gas tube in order to insulate the fluid during transport from the capital unit and prevent a drop in temperature. This further allows the capital unit to be moved away from the patient.

Additionally, systems are provided which allow the vapor transfer unit to be placed farther away from the patient. The use of a water jacket around the gas tubing from the vapor transfer unit to the patient prevents cooling of the gas and condensation. Patient comfort is increased by moving the vapor transfer unit away from the body, resulting in lowered sensation of heat from the vapor transfer unit and increased range of motion during therapy.

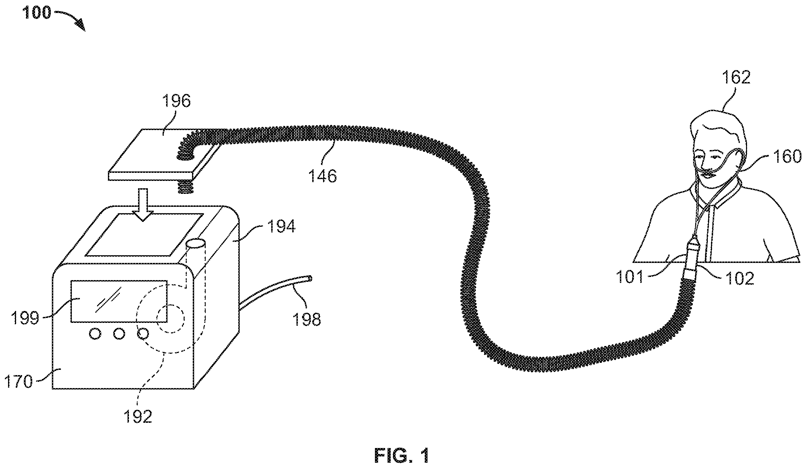

FIG. 1 shows an illustrative respiratory therapy system including capital unit 170, axial flow humidifier 101, and cannula 160 for delivery of humidified gas to patient 162. Capital unit 170 includes blower 192, heater 194, pump 196, and gas supply 198. First gas passageway 146 is connected to capital unit 170 and gas and heated fluid are provided through first gas passageway 146 and first fluid passageway (not shown) within first gas passageway 146. First gas passageway 146 may act as an insulator for the fluid in the first fluid passageway (not shown). The gas and fluid travel, e.g., through medical grade tubing, from capital unit 170 to humidifier 101 where the gas and fluid pass into body 106 where the heated fluid interacts with the gas through a nonporous membrane (not shown), heating and humidifying the gas. The heated and humidified gas is then provided to patient 162 using cannula 160 or other means of therapeutic delivery of gas.

Capital unit 170 includes blower 192 to propel the gas through first gas passageway 146 to body 102. The nonporous membrane may be comprised of a plurality of fibers. The large diameter of the fibers of the nonporous membrane (not shown) in body 102, through which the gas moves, allows for use of blower 192. Small diameter fiber membranes typically require a high pressure air source, such as building air to push the gas through the membrane. Advantageously, use of a blower instead of a high pressure gas source reduces noise associated with the therapy while maintaining a high flow rate. Additionally, capital unit 170 employing blower 192 is more portable, allowing for home use or use in other environments in which room air or other high pressure sources are not available. Blower 192 may also be used with oxygen from a portable tank attached to gas supply 198 in home use scenarios. The gas source may alternatively or additionally be a gas blender, mechanical ventilator, high flow therapy system, oxygen concentrator or oxygen tank. The humidifier can also be used with a continuous positive airway pressure ("CPAP") or bubble CPAP system.

The gas is provided to first gas passageway 146 from gas supply 198 and is propelled by blower 192. The gas and fluid may be heated at heater 194 in capital unit 170 prior to transport to humidifier 101. The fluid is propelled through first fluid passageway (not shown) by pump 196. Pump 196 may be disposable for ease of use in patient care settings and may be inserted into capital unit 170 before use. The heated gas and fluid are transported from capital unit 170 to humidifier 101 where the heated fluid humidifies the gas. The gas is then provided to patient 162 through cannula 160.

Humidifier 101 is positioned proximal to patient 162 to reduce the distance over which the heated and humidified gas travels from humidifier 101 to reach patient 162. The heated gas and the heated fluid travel from capital unit 170 to humidifier 101 over a length of tubing before the gas is humidified. This prevents condensation of moisture from the gas over a long length of tubing and also reduces the length of tubing over which the heated and humidified gas moves from humidifier 101 to patient 162 over which the gas can cool. The heated gas does not need to be insulated during transport between capital unit 170 and humidifier 101 because as it does not contain moisture, there is no risk of condensation occurring in first gas passageway 146 due to heat loss. Transport of the gas between capital unit 170 and humidifier 101 without the need for a water jacket or other insulation mechanism prevents a drop in the pressure of the gas. Thus, by moving the location of humidifier 101 proximate to patient 162, the pressure of the gas is maintained while the amount of condensation that occurs during operation is reduced and the need for additional systems to control condensation is reduced. Use of axial flow humidifier 101 proximate to patient 162 allows patient 162 to comfortably and safely receive respiratory therapy.

Capital unit 170 may also include user-input interface 199 which allows a user to select values associated with the therapy, such as temperature and humidity regulation, flow rate, and timers, as well as to turn the unit on and off. In some embodiments, capital unit 170 may include control circuitry. Control circuitry may receive data via an input/output path from one or more sensors (e.g., that measure flow rate, temperature, humidity, etc.). Control circuitry may be used to send and receive commands (e.g., to operate the humidifier). The control circuitry may be based on any suitable language and/or operating system and may execute instructions for the humidifier that are stored in memory. A patient may also send instructions to the control circuitry (e.g., instructing the capital unit to deliver gas) using any suitable user interface, such as a keypad, keyboard, voice command, etc. Capital unit 170 may also include a display and/or speakers that may be provided as stand-alone devices or integrated with other elements of capital unit 170. For example, the display and/or speakers may issue alerts on a current status or measurement (e.g., flow rate, temperature, humidity, etc.).

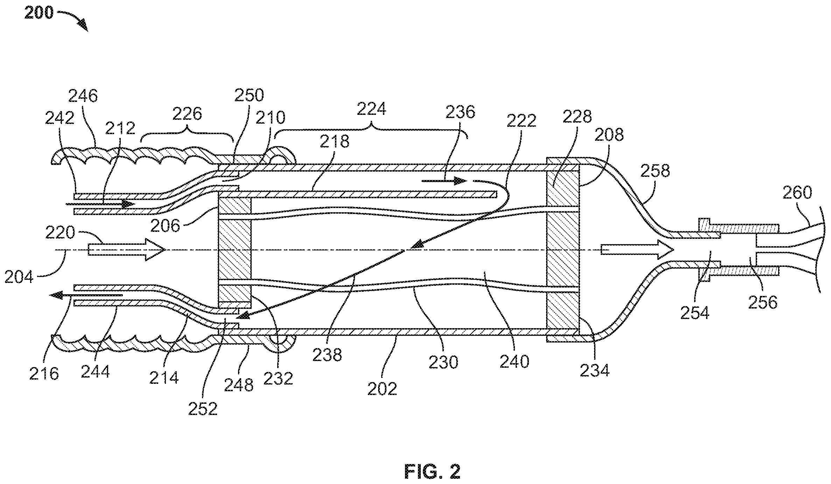

FIG. 2 shows an illustrative axial flow humidifier 200 for humidifying breathing gas for delivery to a proximal patient. The axial flow humidifier includes body 202 having first longitudinal axis 204, from first face 206 to second face 208; first lumen 210 having aperture 222; second lumen 214; and first nonporous membrane 218 and second nonporous membrane 230, first and second nonporous membranes (218, 230) having a bonded connection at first face 206 and at second face 208. First lumen 210 is designed to allow a fluid to enter body 202 at fluid entrance path 212. Fluid enters body 202 into first lumen 210 at fluid entrance path 212 and travels through first lumen 210 in first fluid direction 236 to aperture 222 of first lumen 210 at which point the fluid exits first lumen 210 into body 202 and interacts with first and second nonporous membranes (218, 230). Upon exiting first lumen 210, in some implementations, the fluid may change flow direction to second fluid flow direction 238 to move toward second lumen 214 and fluid exit pathway 116. As the fluid interacts with first and second nonporous membranes (218, 230), the fluid humidifies the gas passing through first and second nonporous membranes (218, 230). The gas continues through first and second nonporous membranes (218, 230) along the length of body 202 and exits body 202 at second face 208.

The fluid is transported to body 102 through first fluid passageway 242. The fluid is heated at capital unit (e.g., capital unit 170 (FIG. 1)) and transported through first fluid passageway 242 which may be plastic medical tubing. The fluid enters body 201 at first end cap 248 where first lumen 210 is joined to first fluid passageway 144 at first end cap aperture 250. The heated fluid enters body 202 through first lumen 210 and exits first lumen 210 at aperture 222. The fluid encounters fiber-like first and second nonporous membranes (218, 230) within body 202. Gas flows from capital unit (not shown) through first gas passageway 246, through first end cap 248 located at first face 206 and into first and second nonporous membranes (218, 230). Gas travels through first and second nonporous membranes (218, 230) along the length of body 202 in the direction of first longitudinal axis 204. The gas is humidified by the fluid as the gas passes through first and second nonporous membranes (218, 230) and is entrained in the gas flow. The gas may achieve a humidity between 25 mg/L to 45 mg/L, or any other suitable humidity. In some implementations a preferred humidity is 35 mg/L. The gas exits body 202 at second face 208. The humidified gas flows through cannula adapter 258 and into cannula 260 for delivery to the patient.

First and second nonporous membranes (218, 230) may be hollow fiber-like membranes through which the gas flows from first face 206 of humidifier body 202 to second face 208. Membranes with a relatively large inner diameter, such as 0.65 mm, may be used in order to allow gas to pass more easily through the membranes. The large membrane diameters allow the use of a blower for portability of the respiratory therapy system. Large fiber diameter also decreases the cost of manufacture by extrusion because fibers of large diameter have greater surface area per unit length, so fewer fibers may be necessary to achieve the same flow and humidification of gas.

First and second nonporous membranes (218, 230) may additionally have thinner walls, allowing for more precise temperature control of the gas. The gas passing through a thin-walled fiber is heated and humidified by the fluid to achieve a temperature similar to the humidifying fluid's. The walls of the nonporous membranes may be 35/1000 mm, 40/1000 mm, 50/1000 mm, for example; however, vapor pressure will need to be increased with increasing thickness of the wall. A nonporous membrane fiber with a 2 mm outer diameter may have a wall with thickness 0.1 mm. Because of the large diameter fiber and thin wall, the pressure drop of the gas flowing through the nonporous membranes to be heated is only 0.01 psi, which allows the system to operate using a blower rather than room air. Fibers having diameters larger or smaller may also be used in the system, for example, nonporous fibers with diameters from 0.5 mm to 4 mm. In some embodiments, body 101 may house fewer nonporous membranes as the larger surface area of the larger diameter fibers maintains the desired surface area of the nonporous membranes. Additionally, or alternatively, the size of the body may be increased to accommodate more larger diameter nonporous membranes and/or to ensure a desired surface area of the nonporous membranes.

After the fluid has moved from first lumen 210 through body 202 and humidified the gas passing through first and second nonporous membranes (218, 230), the fluid travels toward first face 206 and exits body 202 along fluid exit path 216 at second lumen 214. The fluid passes through first end cap 248 at first face 206 at second aperture 252 of first end cap 248 and into second fluid passageway 244. First and second fluid passageways (242, 244) are, in some implementations, contained within first gas passageway 246. This protects first and second fluid passageways (242, 244) from being kinked or dislodged from the body 202 while also insulating the heated fluid with the air surrounding first and second passageways (242, 244). Moreover, the likelihood that first and second fluid passageways (242, 244) become tangled or damaged is reduced by housing them inside first gas passageway 246.

As the gas passes through first and second nonporous membranes (218, 230), it is humidified by the fluid moving through body 202 past first and second nonporous membranes (218, 230). In some implementations first and second nonporous membranes (218, 230) may be manufactured as a water-absorbing plastic which allows water or other fluids to absorb into the plastic and pass through to the interior of the membrane. A nonporous membrane will allow a fluid to pass through but may be constructed so as to inhibit the passage of bacteria or other harmful substances into the gas on the interior of the membrane. The fluid passes through first and second nonporous membranes (218, 230) into the interior of the membrane and is entrained into the gas flow. The humidified gas passes out the end of first and second nonporous membranes (218, 230) at second face 208 and into second gas passageway 254 connected to cannula 260 attached to body 202 at cannula adapter 258.

The nonporous membrane allows the heated fluid to interact with the gas inside the membrane in order to heat and humidify the gas. The nonporous membrane may be permeable to gas, but impermeable to liquid. Thus, water vapor is permitted to permeate the membrane, while liquid water is not. Water vapor may penetrate the membrane and become entrained in the gas flow, thus humidifying and heating the gas. Furthermore, the membrane is nonporous and as such there are no direct openings in the membrane through which liquid water may travel.

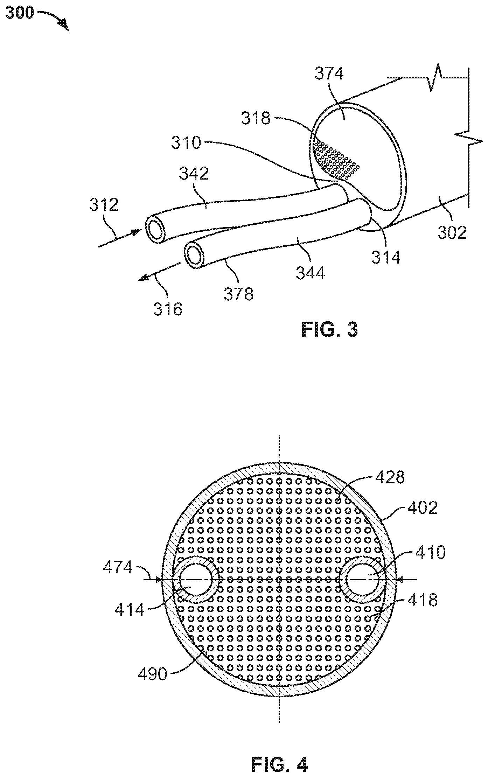

FIG. 3 shows an illustrative view of first and second fluid passageways (342, 344) attached to the exterior of axial flow humidifier 200 of FIG. 2. First and second fluid passageways (342, 344) are attached to body 302 of the axial flow humidifier at first face 306. First and second fluid passageways (342, 344) may be connected to first face 306 in a variety of arrangements, including first fluid passageway 342 next to second fluid passageway 344 on one side of body 302 at first face 306 in the direction of longitudinal axis 304 as shown in this view. First and second fluid passageways (342, 344) may be connected to body (302) at opposite points on first face 306 or in any other suitable configuration. Connecting first and second fluid passageways (342, 344) to body 302 at first face 306 allows axial flow humidifier 300 to maintain a streamlined and non-bulky shape as compared to humidifiers that attach fluid lines at a side of body 302. The streamlined shape increases patient comfort by decreasing bulk that may be located on or near the patient during treatment. The connection of first and second fluid passageways (342, 344) at first face 306 of body 302 further decreases the likelihood that first and second fluid passageways (342, 344) will be tangled, kinked or accidentally disconnected during use. Additionally, attaching first and second fluid passageways (342, 344) at first face 306 of body 302 does not require lateral ports on the sides of body 302, allowing body 302 to be easily manufactured by extrusion.

The ends of nonporous membranes (318, 330) are visible at first face 306 of body 302. Nonporous membranes (318, 330) are arranged in body 302 extending from first face 306 to second face 308. Nonporous membranes (318, 330) are constructed as hollow fibers through which gas may be passed.

During use, fluid passes into body 302 through first fluid passageway 342 along fluid entrance path 312. The fluid exits body 302 along fluid exit path 316, exiting body 302 into second fluid passageway 344. First and second fluid passageways (342, 344) may have an inner diameter of between 1 mm and 8 mm, or any other suitable size. Fluid passageways (342, 344) allow the fluid to be transported to and from the humidifier. After use, fluid may be returned to capital unit (not shown) to be recycled or disposed of.

FIG. 4 shows an illustrative cross-sectional view of an axial flow humidifier. The cross-sectional view includes first lumen 410 opposite second lumen 414 located at wall 490 of body 402, and the end view of first nonporous membrane 418. Body 402 may have internal diameter 474 sufficient to encapsulate a plurality of nonporous membranes (e.g., 20, 50, 100, 1000, or any other suitable number of fibers). Because the nonporous fibers have a larger diameter, the pressure drop as gas travels through the fiber is decreased and the gas can be propelled through the fiber by a lower pressure blower as the gas pressure will be maintained throughout the humidifier.

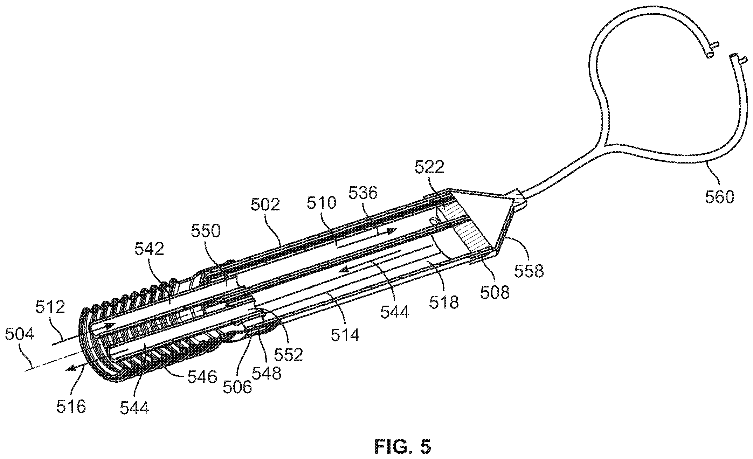

FIG. 5 shows an illustrative view of the axial flow humidifier 500 in connection with a patient delivery cannula. In use, axial flow humidifier 500 is placed proximal to a patient in order to humidify heated gas for delivery to the patient without condensation of the humidified gas in a lengthy delivery tube. The gas flows through first gas passageway 546 and into body 502 to flow through nonporous membranes 518 from first face 506 to second face 508. The liquid is transported to body 502 through first liquid passageway 542 contained in first gas passageway 546. At first face 506, the liquid enters body 502 at first end cap 548 through first aperture 550 of end cap 548, which connects first liquid passageway 542 to first lumen 510. Within body 502, the fluid exits first lumen 510 at aperture 522 of the first lumen 510. The fluid humidifies the gas passing through nonporous membranes (518, 530) and exits body 502 by entering second lumen 514, traveling toward first face 306 and passing out through second aperture 552 in end cap 548 into second fluid passageway 544, also contained in first gas passageway 546. Placement of first and second fluid passageways (542, 544) within first gas passageway 546 protects first and second fluid passageways (542, 544) from kinking or becoming dislodged from body 502. This placement additionally decreases the amount of heat loss experienced by the fluid as it is transported through first fluid passageway 544, as first fluid passageway 544 is insulated by the surrounding gas in first gas passageway 546. Any heat lost from the fluid as it moves through first fluid passageway 544 within first gas passageway 546 serves to heat the gas in first gas passageway 546, rather than being lost to ambient air if first fluid passageway 544 were not insulated by the gas in first gas passageway 546.

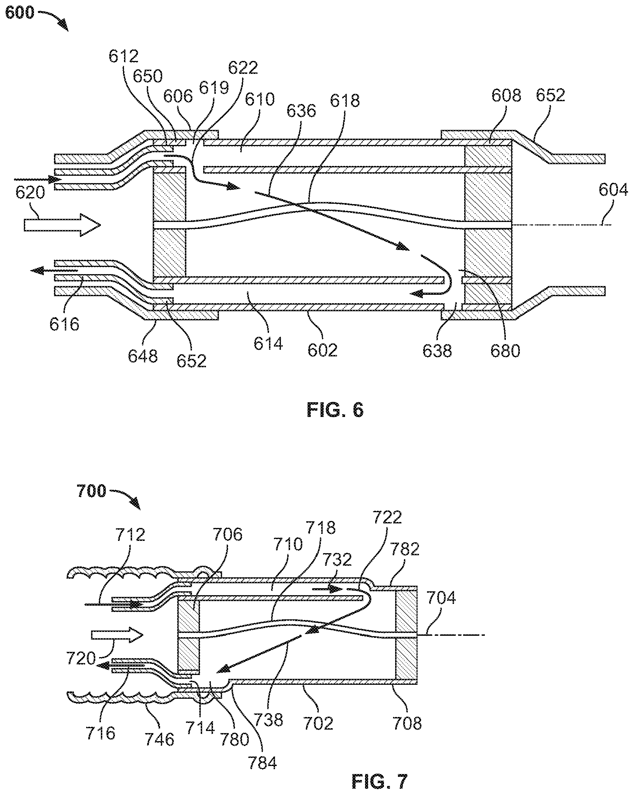

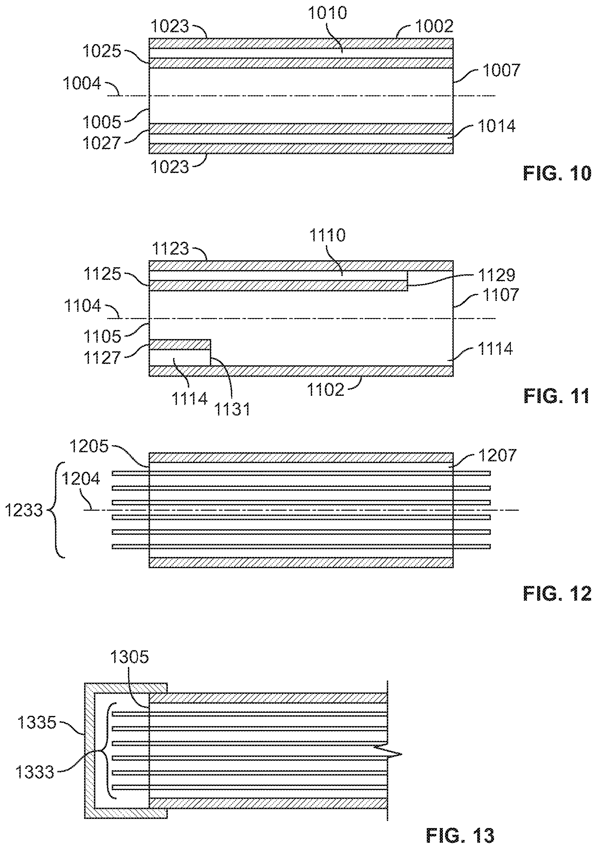

FIG. 6 shows an illustrative axial flow humidifier 600 having fluid flow in a direction not counter to the gas flow. The axial flow humidifier 600 shown in FIG. 6 shows an alternative configuration from the axial flow humidifier 200 of FIG. 2. Fluid flows through first fluid passageway 642 to first end cap 648 located near first face 606 where first fluid passageway 642 joins body 602 at first aperture 650 in first end cap 648. The fluid flows into body 602 and into first lumen 610. In the configuration shown in FIG. 6, aperture 622 of first lumen 610 is located proximal to first face 606 such that fluid enters body 602 and exits first lumen 610 shortly thereafter. Aperture of the first lumen 610 may be produced during manufacture by drilling through a side of body 602 to produce aperture 619 in body 602 as well as aperture 622 of first lumen 610 as discussed in reference to FIGS. 10-16. For example, after body 602 is extruded, a side of body 602 may be penetrated to create aperture 619 and then, as the drilling continues, create aperture 622. Aperture 619 may then be sealed when first end cap 648 is attached to body 602 or through the application of a suitable sealing material to aperture 619.

The fluid enters body 602 with a flow direction substantially parallel to longitudinal axis 604 of body 602. The fluid leaves first lumen 610 and flows past first nonporous membrane 618, humidifying gas flowing through first nonporous membrane 618. The fluid may flow through body 602 generally in a direction similar to the first direction of fluid flow 636 as it moves toward second face 608. Second lumen 614 extends from first face 606 and has aperture of the second lumen 680 proximal to second face 608. The fluid, once exited from first lumen 610, moves through body 602 and into second lumen 614 toward first face 606 according to second direction of fluid flow 638, which is counter to first direction of fluid flow 636. A liquid return path through body 602 which is counter to the first direction of fluid flow may be more efficient when the humidifier is oriented in an upright position such as on a bed rail or on an upright patient. The gas may pass through body 602 in the first nonporous membrane 618 and out through second face 608. The gas may then travel along second gas passageway 652 to a patient interface (not shown).

FIG. 7 shows an illustrative axial flow humidifier 700 having an alternative fluid flow return aperture. The fluid flows along first fluid passageway 712 to first face 706 where first fluid passageway 742 joins with first lumen 710 to enter body 702. In the configuration of FIG. 7, first lumen 710 extends along the length of body 702, and aperture 722 of first lumen 710 is located proximal to second face 708. First lumen 710 is shown as an addition to body 702 of humidifier ends at first shoulder 782 proximal to second face 708. The fluid exits first lumen 710 at aperture 722 of first lumen 710 and passes first nonporous membrane 718 where heated fluid humidifies gas flowing through first nonporous membrane 718. The fluid may flow through first lumen 710 having first direction of fluid flow 736 parallel to longitudinal axis 704, and after exiting first lumen 710, the fluid may change direction and flow through body 702 and past nonporous membrane 718 generally in second direction of fluid flow 738. The fluid may then enter second lumen 714. Second lumen 714 is shown having aperture 780 of second lumen 714 proximal to first face 706. Second lumen 714 is also shown as an addition to body 702 and has shoulder 784 where second lumen 714 joins body 702. In some implementations, second lumen 714 and first lumen 710 may be manufactured as separate components which are joined to body 702. In other implementations body 702 and first and second lumens (710, 714) may be manufactured as a single unit.

The alternative fluid flow paths through the body of the axial flow humidifier illustrated in FIGS. 6 and 7 provides pathways for fluid flow from aperture (622, 722) of first lumen (610, 710) through body (602, 702) of the humidifier and past nonporous membranes (618, 718) to aperture (680, 780) of second lumen (614, 714) for return. These and any other suitable pathways from first lumen (610, 710) to second lumen (614, 714) are contemplated for use in a humidifier, as the optimal fluid pathway may be changed by the positioning and orientation of the humidifier for therapeutic use.

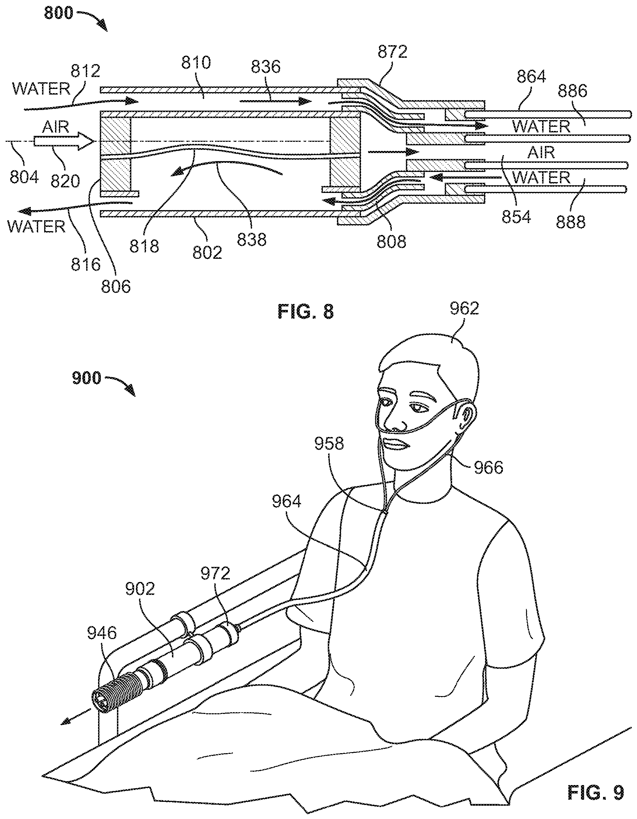

FIG. 8 shows an illustrative axial flow humidifier 800 having extended fluid and gas pathways for use at a distance from the patient. A heated fluid enters body 802 along fluid entrance path 812 passing into first lumen 810 at first face 806. The fluid flows along first lumen 810 to second face 808 where the fluid flows out through second end cap 872 and into first extended fluid passageway 886. Fluid may flow through first extended fluid passageway 886 in one direction and flow back to body 802 in second extended fluid passageway 888. Once in body 802, the fluid may flow past first lumen 810 carrying the gas and humidify the gas. The fluid may then flow in second fluid flow direction 838 generally counter to first fluid flow direction 836 toward first face 806. The fluid may finally pass out of body 802 along fluid exit path 816. The gas enters body 802 in a direction parallel to longitudinal axis of the body 804 and travels through first nonporous membrane 818 where it is humidified by the fluid. The gas passes through first nonporous membrane 818 and out second face 808 at second end cap 872 and into second gas passageway 854.

First extended fluid passageway 886 and second extended fluid passageway 888 may be configured to surround second gas passageway 854 such that the gas in second gas passageway 854 is insulated by the water in first and second extended fluid passageways (886, 888). This decreases heat loss of the gas as it moves through second gas passageway 854 to the patient and minimizes condensation of the humidified gas. The application of first and second extended fluid passageways (886, 888) to surround and insulate second gas passageway 854 may be referred to as a water jacket. In some implementations an additional coating or wrapping of second gas passageway 854 and first and second extended fluid passageways (886, 888) may be used to further insulate the humidified gas. A water jacket allows humidifier 800 to be placed at a greater distance from the patient without increasing the condensation of the humidified gas over the distance from the humidifier to the patient by maintaining a constant temperature. For example, the humidifier with a water jacket may be placed on a bed rail or a stand at a distance from the patient without increased risk of condensation. This increases patient comfort as the humidifier does not need to be in direct contact with the patient.

FIG. 9 shows an illustrative respiratory therapy delivery system including axial flow humidifier 800, water jacket 964 and cannula 966 for delivery of humidified gas to patient 962. The illustrated system includes a humidifier such as that depicted in FIG. 8 in an exemplary use in a therapy delivery system. Humidifier body 902 is connected to a capital unit (not shown) by gas passageway 946 and internal to gas passageway 946 and first and second fluid passageways (not shown). The capital unit provides gas as well as heated fluid through the passageways. Gas passageway 946 and fluid passageways enter body 902 where the gas is heated and humidified by the fluid. The heated and humidified gas passes out of body 902 and into second gas passageway 954 at second end cap 972. Second gas passageway 954 is insulated by the heated fluid which flows through first and second extended fluid passageways (not shown) surrounding second gas passageway which act as water jacket 964. The heated and humidified gas is administered to patient 962 through cannula 966 connected to second gas passageway 954 at cannula adapter 958, which may dramatically decrease in cross-sectional area in order to increase the flow rate of the gas and minimize condensation in the cannula. The insulated gas passageway maintains a constant temperature and decreases condensation of the heated humidified gas as it travels from body 902 to patient 962. Body 902 can thus be attached at a bed rail or otherwise disposed not on the patient. Body 902 may be disposed at a distance from the patient of 3 feet, 2.5 feet, 2 feet, 1 foot or less without a problematic drop in pressure or temperature. Patient comfort and range of movement are increased by moving the humidifier proximal to the patient but not in contact with the patient.

FIGS. 10-16 illustrate a method for manufacturing an axial flow humidifier by extrusion. FIG. 10 shows an illustrative extruded body 1002 of an axial flow humidifier such as those shown in FIGS. 1, 2, 3, 5, 6, 7, 8 or 9. Body 1002 is extruded as a hollow cylinder having outer wall 1023, which may be circular in cross-section, and first lumen 1010 and second lumen 1014 are formed along interior of the outer wall 1023 from first side 1005 to second side 1007. First and second lumens (1010, 1014) are parallel to longitudinal axis 1004 of body 1002. Each of first and second lumens (1010, 1014) are formed by outer wall 1023 and first or second interior wall (1025, 1027). In some implementations first and second lumens (1010, 1014) may additionally share a wall (e.g., as shown in FIG. 3). Body 1002 and first and second lumens (1010, 1014) may be extruded as a single extrusion.

In FIG. 11, first and second lumens (1110, 1114) of extruded body 1102 are cut at the interior to cylindrical body 1102 at first and second interior lumen walls (1125, 1127). First lumen 1110 is cut at first position 1 129 at first interior lumen wall 1125 such that the interior of first lumen 1110 is now exposed to the interior of body 1102. Second lumen 1118 is cut at second position 1131 of second interior lumen wall 1127, exposing the interior of second lumen 1118 to the interior of body 1102. First position 1129 is aperture of the first lumen 1122 through which fluid flows into body 1102 during later use. In some implementations, the interior of first lumen 1110 is exposed to the interior of body 1102 by forming a hole which penetrates through the outside of body 1102 and through first lumen 1110, for example as shown in FIG. 6. The hole may later be covered by the end cap (not shown), or otherwise sealed in order to close the interior of body 1102 from the outside.

In FIG. 12, nonporous membranes 1233 are inserted into the interior of body 1202 such that the nonporous membranes fill the space and are parallel to longitudinal axis 1204 of body 1202 and extend from first side 1205 to second side 1207. Bundle of nonporous membranes 1233 may be inserted with a shim, funnel, paper or plastic sheet or by any other suitable means.

FIG. 13 shows body 1302 filled with nonporous membranes 1333 with end cap 1335 in place at first side 1305. Though not shown, a second end cap is placed on the end of body 1302 at a second side (not shown) in order to completely enclose the interior of body 1302.

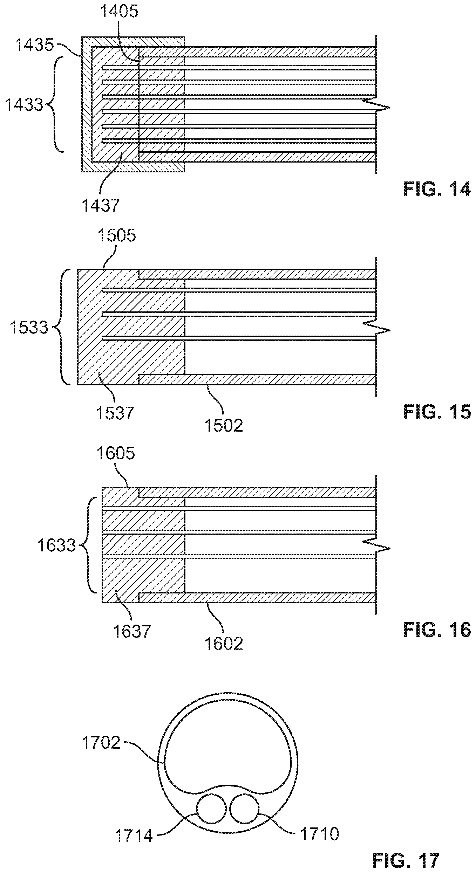

FIG. 14 shows the end of body 1402 having end cap 1435 in place at first side 1405 after potting material 1437 has been injected into body 1402 during centrifugation. Potting material 1437 is injected through an injection site (not shown) at an end or side of body 1402. Potting material may be injected through the hole in body 1402 and first lumen as shown in FIG. 6. After injection, body is centrifuged to distribute potting material 1437 at the ends of body 1402 near first side 1405 and second side 1407. Potting material serves to bond the ends of nonporous membranes together to hold them in place. The potting material may be PET, PETG, PETE, a polycarbonate, and/or any material suitable for bonding the non-porous membranes to each other and in place.

FIG. 15 shows body 1502 containing nonporous membranes 1533 bonded together by potting material 1437 with the end cap removed. As shown by FIG. 15 after removal of end cap 1435, potting material 1437 acts to hold nonporous membranes 1533 in a shape formed by end cap 1435. Furthermore, nonporous membranes 1533 extend beyond body 1402, allowing for easy access to cut potting material 1437 and nonporous membranes 1533 along the cross-section of body 1402.

In FIG. 16, the end of body 1602 where nonporous membranes 1633 are bonded together by potting material 1637 has been cut to expose the hollow ends of nonporous membranes 1633. At least a portion of potting material 1637 remains such that bundle of nonporous membranes 1633 remains bonded together.

Manufacture of the axial flow humidifier by extrusion is cost-effective and efficient. The manufacture process is simple and allows the humidifier to be produced with a seamless and sleek cylindrical shape for patient comfort and ease of use. Use of end caps which connect fluid and gas to the humidifier further adds to the sleek design and is compatible with manufacture of the humidifier body by extrusion. The end cap may allow the first and second fluid passageways to be disposed within the first gas passageway.

FIG. 17 shows an illustrative cross-sectional view of an axial flow humidifier having two lumens (1710, 1714) on one side of the cross-sectional body. The cross-sectional view illustrates the hollow openings of nonporous membranes 1733 through which gas may pass. The cross-sectional view includes first lumen 1710 located next to second lumen 1714 at the wall of body 1790, and the end view of first nonporous membrane 1718. The placement of first and second lumens (1710, 1714) may depend on manufacturing considerations. Alternatively, arrangements of first and second lumen (1710, 1714), including those illustrated in FIGS. 4 and 17, may provide optimal flow for various orientations and placements of the humidifier on or near a patient.

The foregoing is merely illustrative of the principles of the disclosure, and the systems, devices, and methods can be practiced by other than the described embodiments, which are presented for purposes of illustration and not of limitation. It is to be understood that the systems, devices, and methods disclosed herein, while shown for use in high flow therapy systems, may be applied to systems, devices, and methods to be used in other ventilation circuits.

Variations and modifications will occur to those of skill in the art after reviewing this disclosure. The disclosed features may be implemented, in any combination and subcombination (including multiple dependent combinations and subcombinations), with one or more other features described herein. The various features described or illustrated above, including any components thereof, may be combined or integrated in other systems. Moreover, certain features may be omitted or not implemented.

Examples of changes, substitutions, and alterations are ascertainable by one skilled in the art and could be made without departing from the scope of the information disclosed herein.

## Claims

1. A system (200) for humidifying gases, the system (200) comprising:
a body (202), extending along a first longitudinal axis (204) from a first face (206) to a second face (208);
a first lumen (210), at the first face (206), through which fluid enters the body (202);
a second lumen (214), at the first face (206), through which the fluid exits the body (202); and
a first non-porous membrane (218), inside the body (202), extending from the first face (206) to the second face (208), wherein gas moving through the first non-porous membrane (218) is humidified by the fluid, wherein the first non-porous membrane (218) is permeable to the gas, and **characterized in that** the first non-porous membrane (218) comprises a plurality of large-diameter fibers, each of the plurality of fibers having a diameter of about 0.5 millimeters to about 2.0 millimeters.

2. The system (200) of claim 1, wherein the first lumen (210) and the second lumen (214) are substantially parallel to the first longitudinal axis (204).

3. The system (200) of claim 1, wherein the first lumen (210) and the second lumen (214) have different lengths.

4. The system (200) of any of claims 1-3, wherein the first lumen (210) includes an aperture (222) allowing the fluid to contact the first non-porous membrane (218) while inside the body (202).

5. The system (200) of any of claims 1-4, wherein each of the plurality of fibers is a hollow fiber defining a fiber lumen through which the gas flows.

6. The system (200) of any of claims 1-5, further comprising a second non-porous membrane (230), inside the body (202), extending from the first face (206) to the second face (208), wherein the second non-porous membrane (230) is bonded to the first non-porous membrane (218) at the first face (206) and the second face (208).

7. The system (200) of any of claims 1-6, wherein the fluid moves in a first direction and the gas moves in a second direction that is substantially parallel to the first direction.

8. The system (200) of any of claims 1-7, further comprising:
a first fluid passageway (242) connecting to the first lumen (210) through which the fluid is delivered to the body (202);
a second fluid passageway (244) connecting to the second lumen (214) through which the fluid is removed from the body (202); and
a first gas passageway (246) through which the gas is delivered to the body (202), wherein the first fluid passageway (242) and the second fluid passageway (244) are housed within the first gas passageway (246).

9. The system (200) of claim 8, further comprising a first end cap (548) connecting the body (202) to the first gas passageway (246) at the first face (206), wherein the first fluid passageway (242) and the second fluid passageway (244) pass through the first end cap (548).

10. The system (200) of any of claims 8-9, further comprising a second gas passageway (254) at the second face (208), wherein the second gas passageway (254) delivers the humidified gas from the body (202) to a patient.

11. The system (200) of claim 10, further comprising a water jacket (964) located at the second gas passageway (254), wherein the water jacket (964) warms the humidified gas as the humidified gas is delivered from the body (202) to the patient.

12. A method of manufacturing the system (200) of any of claims 1-11.

## Patentansprüche

1. System (200) zum Befeuchten von Gasen, wobei das System (200) Folgendes umfasst:
einen Körper (202), der sich entlang einer ersten Längsachse (204) von einer ersten Fläche (206) zu einer zweiten Fläche (208) erstreckt;
ein erstes Lumen (210) an der ersten Fläche (206), durch das Fluid in den Körper (202) eintritt;
ein zweites Lumen (214) an der ersten Fläche (206), durch welches das Fluid aus dem Körper (202) austritt; und
eine erste nichtporöse Membran (218) innerhalb des Körpers (202), die sich von der ersten Fläche (206) zu der zweiten Fläche (208) erstreckt, wobei Gas, das sich durch die erste nichtporöse Membran (218) bewegt, durch das Fluid befeuchtet wird, wobei die erste nichtporöse Membran (218) durchlässig für das Gas ist, und **dadurch gekennzeichnet, dass**
die erste nichtporöse Membran (218) eine Vielzahl von Fasern mit großem Durchmesser umfasst,
wobei jede aus der Vielzahl von Fasern einen Durchmesser von etwa 0,5 Millimetern bis etwa 2,0 Millimetern aufweist.

2. System (200) nach Anspruch 1, wobei das erste Lumen (210) und das zweite Lumen (214) im Wesentlichen parallel zu der ersten Längsachse (204) sind.

3. System (200) nach Anspruch 1, wobei das erste Lumen (210) und das zweite Lumen (214) unterschiedliche Längen aufweisen.

4. System (200) nach einem der Ansprüche 1-3, wobei das erste Lumen (210) eine Öffnung (222) beinhaltet, die dem Fluid ermöglicht, die erste nichtporöse Membran (218) zu kontaktieren, während es sich innerhalb des Körpers (202) befindet.

5. System (200) nach einem der Ansprüche 1-4, wobei jede aus der Vielzahl von Fasern eine Hohlfaser ist, die ein Faserlumen definiert, durch welches das Gas strömt.

6. System (200) nach einem der Ansprüche 1-5, ferner umfassend eine zweite nichtporöse Membran (230) innerhalb des Körpers (202), die sich von der ersten Fläche (206) zu der zweiten Fläche (208) erstreckt, wobei die zweite nichtporöse Membran (230) an der ersten Fläche (206) und der zweiten Fläche (208) an die erste nichtporöse Membran (218) gebunden ist.

7. System (200) nach einem der Ansprüche 1-6, wobei sich das Fluid in eine erste Richtung bewegt und sich das Gas in eine zweite Richtung bewegt, die im Wesentlichen parallel zu der ersten Richtung ist.

8. System (200) nach einem der Ansprüche 1-7, ferner umfassend:
einen ersten Fluiddurchlass (242), der mit dem ersten Lumen (210) verbunden ist, durch welches das Fluid an den Körper (202) abgegeben wird;
einen zweiten Fluiddurchlass (244), der mit dem zweiten Lumen (214) verbunden ist, durch welches das Fluid aus dem Körper (202) entfernt wird; und
einen ersten Gasdurchlass (246), durch welches das Gas an den Körper (202) abgegeben wird, wobei sich der erste Fluiddurchlass (242) und der zweite Fluiddurchlass (244) innerhalb des ersten Gasdurchlasses (246) befinden.

9. System (200) nach Anspruch 8, ferner umfassend eine erste Endkappe (548), die den Körper (202) mit dem ersten Gasdurchlass (246) an der ersten Fläche (206) verbindet, wobei der erste Fluiddurchlass (242) und der zweite Fluiddurchlass (244) die erste Endkappe (548) durchlaufen.

10. System (200) nach einem der Ansprüche 8-9, ferner umfassend einen zweiten Gasdurchlass (254) an der zweiten Fläche (208), wobei der zweite Gasdurchlass (254) das befeuchtete Gas von dem Körper (202) an einen Patienten abgibt.

11. System (200) nach Anspruch 10, ferner umfassend einen Wassermantel (964), der sich an dem zweiten Gasdurchlass (254) befindet, wobei der Wassermantel (964) das befeuchtete Gas erwärmt, während das befeuchtete Gas von dem Körper (202) an den Patienten abgegeben wird.

12. Verfahren zum Herstellen des Systems (200) nach einem der Ansprüche 1-11.

## Revendications

1. Système (200) pour humidifier des gaz, le système (200) comprenant :
un corps (202), s'étendant le long d'un premier axe longitudinal (204) d'une première face (206) à une seconde face (208) ;
une première lumière (210), au niveau de la première face (206), par laquelle le fluide entre dans le corps (202) ;
une seconde lumière (214), au niveau de la première face (206), par laquelle le fluide sort du corps (202) ; et
une première membrane non poreuse (218), à l'intérieur du corps (202), s'étendant de la première face (206) à la seconde face (208), dans lequel le gaz se déplaçant à travers la première membrane non poreuse (218) est humidifié par le fluide, dans lequel la première membrane non poreuse (218) est perméable au gaz, et **caractérisé en ce que** la première membrane non poreuse (218) comprend une pluralité de fibres de grand diamètre, chacune de la pluralité de fibres ayant un diamètre d'environ 0,5 millimètre à environ 2,0 millimètres.

2. Système (200) selon la revendication 1, dans lequel la première lumière (210) et la seconde lumière (214) sont sensiblement parallèles au premier axe longitudinal (204).

3. Système (200) selon la revendication 1, dans lequel la première lumière (210) et la seconde lumière (214) ont des longueurs différentes.

4. Système (200) selon l'une quelconque des revendications 1 à 3, dans lequel la première lumière (210) comporte une ouverture (222) permettant au fluide d'entrer en contact avec la première membrane non poreuse (218) lorsqu'il est à l'intérieur du corps (202) .

5. Système (200) selon l'une quelconque des revendications 1 à 4, dans lequel chacune de la pluralité de fibres est une fibre creuse définissant une lumière de fibre par laquelle le gaz s'écoule.

6. Système (200) selon l'une quelconque des revendications 1 à 5, comprenant en outre une seconde membrane non poreuse (230), à l'intérieur du corps (202), s'étendant de la première face (206) à la seconde face (208), dans lequel la seconde membrane non poreuse (230) est liée à la première membrane non poreuse (218) au niveau de la première face (206) et de la seconde face (208) .

7. Système (200) selon l'une quelconque des revendications 1 à 6, dans lequel le fluide se déplace dans une première direction et le gaz se déplace dans une seconde direction qui est sensiblement parallèle à la première direction.

8. Système (200) selon l'une quelconque des revendications 1 à 7, comprenant en outre :
un premier passage de fluide (242) se connectant à la première lumière (210) par lequel le fluide est fourni au corps (202) ;
un second passage de fluide (244) se connectant à la seconde lumière (214) par lequel le fluide est retiré du corps (202) ; et
un premier passage de gaz (246) par lequel le gaz est fourni au corps (202), dans lequel le premier passage de fluide (242) et le second passage de fluide (244) sont logés à l'intérieur du premier passage de gaz (246).

9. Système (200) selon la revendication 8, comprenant en outre un premier capuchon d'extrémité (548) connectant le corps (202) au premier passage de gaz (246) au niveau de la première face (206), dans lequel le premier passage de fluide (242) et le second passage de fluide (244) traversent le premier capuchon d'extrémité (548).

10. Système (200) selon l'une quelconque des revendications 8 et 9, comprenant en outre un second passage de gaz (254) au niveau de la seconde face (208), dans lequel le second passage de gaz (254) fournit le gaz humidifié du corps (202) à un patient.

11. Système (200) selon la revendication 10, comprenant en outre une chemise d'eau (964) située au niveau du second passage de gaz (254), dans laquelle la chemise d'eau (964) réchauffe le gaz humidifié lorsque le gaz humidifié est fourni du corps (202) au patient.

12. Procédé de fabrication du système (200) selon l'une quelconque des revendications 1 à 11.
